# EUROPEAN PATENT SPECIFICATION

(11) **EP 3 781 925 B1**
(45) Date of publication and mention of the grant of the patent: **06.12.2023**
(21) Application number: 19723872.8
(22) Date of filing: 15.04.2019
(51) Int. Cl.: B23P 19/00, B23P 19/04, B23P 21/00, G01N 3/04, B23P 19/06, G01N 33/44, G01N 35/00, G01N 35/04

(54) **ASSEMBLING MACHINE AND ASSEMBLY METHOD FOR COUPLING TO TWO SAMPLE-HOLDERS THE ENDS OF AN ELASTOMER SPECIMEN TO BE SUBJECTED TO DYNAMIC MECHANICAL-THERMAL ANALYSIS**
MONTAGEMASCHINE UND MONTAGEVERFAHREN ZUM VERBINDEN DER ENDEN EINER ELASTOMERPROBE, DIE EINER DYNAMISCHEN MECHANISCH-THERMISCHEN ANALYSE UNTERZOGEN WERDEN SOLL, MIT ZWEI PROBENHALTERN
MACHINE D'ASSEMBLAGE ET PROCÉDÉ D'ASSEMBLAGE POUR L'ACCOUPLEMENT À DEUX PORTE-ÉCHANTILLONS DES EXTRÉMITÉS D'UN SPÉCIMEN D'ÉLASTOMÈRE À SOUMETTRE À UNE ANALYSE THERMOMÉCANIQUE DYNAMIQUE

(30) Priority: 19.04.2018 IT 201800004705
(43) Date of publication of application: 24.02.2021
(73) Proprietor: Bridgestone Europe NV/SA, 1930 Zaventem (BE)
(72) Inventor: STRAFFI, Paolo, 00128 Roma (IT); COSSU, Giancarlo, 00128 Roma (IT); CAPALDI, Cristian, 00128 Roma (IT); AGORETTI, Pasquale, 00128 Roma (IT)
(74) Representative: Marchetti, Alessio
(86) International application number: PCT/IB2019/053070
(87) International publication number: WO 2019/202462

(56) References cited:
- WO-A1-2013/004812
- JP-U- S5 635 043
- US-A- 3 885 424
- US-A- 4 606 230
- US-A1- 2014 053 656

## Description

### TECHNICAL SECTOR

This invention relates to an assembling machine and to an assembly method for coupling to two sample-holders the ends of an elastomer specimen to be subjected to dynamic mechanical-thermal analysis.

### STATE OF THE ART

In the study and development of elastomers (the generic term elastomer indicates materials that after a deformation imposed by a deforming force are able to return to approximately the initial shape and size at the end of the applied deforming force) to be used for the construction of tyres, dynamic mechanical/thermal tests are frequently carried out on elastomer specimens of standard shape in such a way as to detect experimentally the mechanical/thermal properties of the elastomers.

For example, there is a frequent use of the so-called dynamic mechanical-thermal analysis (DMTA, also known as dynamic mechanical-thermal spectroscopy), which is a thermal analysis technique used for the study of the viscoelastic properties of elastomers and is obtained by applying to a specimen a deformation oscillating under a given condition (temperature and frequency) and in a given way (traction, compression, shear, bending, etc.).

The mechanical-thermal dynamic analysis is carried out in a measuring machine which comprises a closed chamber at a controlled temperature and a test station that is housed in the closed chamber and is made to apply a predetermined mechanical stress to a specimen. In particular, the dynamic mechanical-thermal analysis can be carried out with elastomer samples previously assembled with appropriate sample-holders to be coupled to the test station arranged in the closed chamber. The elastomer samples have a predefined shape (for example of parallelepiped geometry); at the ends of each sample are fastened two sample-holders, which are provided with respective vices to clamp (and therefore lock) the sample with a predetermined clamping force and are provided with connection members to be fixed to the measuring machine.

Currently, the dimensional verification of the specimens (before measuring a specimen it is necessary to know with precision its dimensions) and subsequent assembly of the sample-holders to the specimens are carried out manually by a laboratory operator; however, this manual intervention slows down the process and above all makes the process less repeatable and less controllable since accidental errors are potentially introduced due to the fact that the laboratory operator can unpredictably make mistakes. In particular, the laboratory operator must measure (at least) the thickness of an elastomer specimen, must assemble the elastomer specimen with the two sample-holders by checking the correct alignment of the specimen with respect to the two sample-holders and by applying a predetermined fixing force, and finally must load the specimen in the measuring machine communicating (typically by means of a real or virtual keyboard) to the control unit of the measuring machine the thickness of the specimen, the identifier of the specimen, and the type of test to be carried out. It is therefore evident that there are many passages wherein the laboratory operator can commit (inadvertently) errors (almost always due to distraction consequent to the continuous repetition of a limited number of operations).

The patent application US2014053656A1 disclosing an assembling machine and assembling method according to the preamble of claims 1 and 14 respectively, describes different types of sample-holders for elastomer specimens to be subjected to dynamic mechanical-thermal analysis.

### DESCRIPTION OF THE INVENTION

The objective of the present invention is to provide an assembling machine and an assembly method for coupling to two sample-holders the ends of an elastomer specimen to be subjected to dynamic mechanical-thermal analysis, which assembling machine and assembly method are free from the drawbacks described above and are, in particular, easy and economic to produce.

According to this invention, an assembling machine according to claim 1 and an assembly method according to claim 14 are provided for coupling to two sample-holders the ends of an elastomer specimen to be subjected to dynamic mechanical-thermal analysis, according to what is stated in the annexed claims.

The dependent claims describe preferred embodiments of the present invention forming an integral part of the present description.

### BRIEF DESCRIPTION OF THE DRAWINGS

The present invention is now described in reference to the attached drawings, which illustrate several non-limiting exemplary embodiments, wherein:
- Figure 1 is a perspective view of an elastomer specimen that must be subjected to dynamic and mechanical-thermal analysis;
- Figure 2 is a perspective view of the specimen of Figure 1 coupled to two sample-holders;
- Figure 3 is a plan view of an assembling machine for the preparation of elastomer specimens and created in accordance with this invention;
- Figures 4, 5 and 6 are respectively a right side view, a left side view and a front view of the assembling machine of Figure 3; and
- Figure 7 is a perspective view, with parts removed for clarity, of a processing station of a mounting unit of the assembling machine of Figure 3.

### PREFERRED EMBODIMENTS OF THE INVENTION

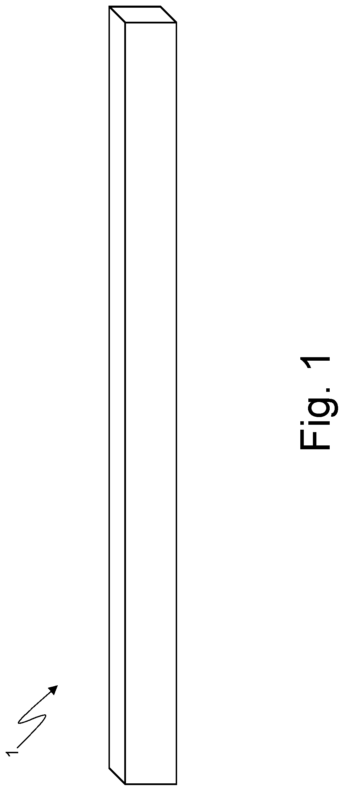

In Figure 1, the numeral 1 denotes in its entirety an elastomer specimen which has a parallelepiped shape and must be subjected to dynamic mechanical-thermal analysis.

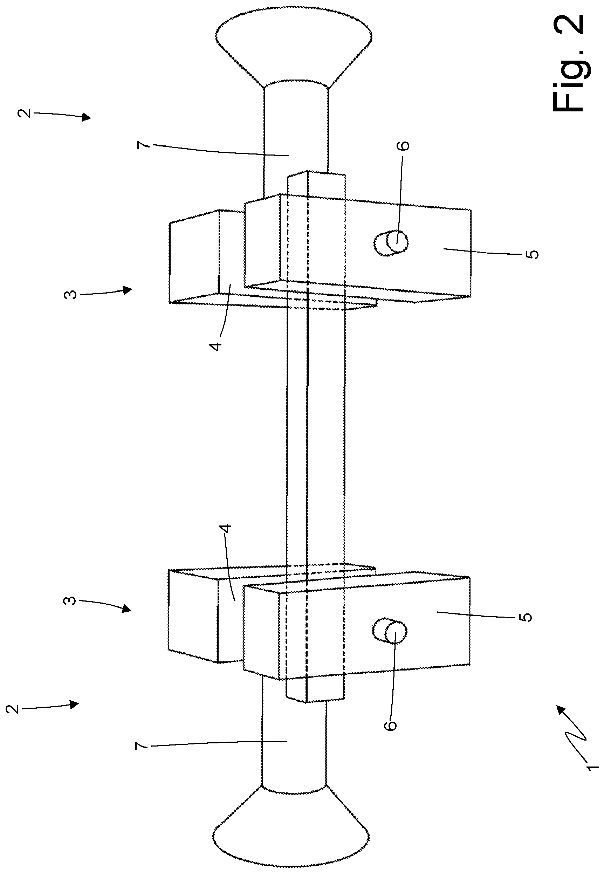

As shown in Figure 2, the specimen 1 must be coupled to two sample-holders 2 which engage the two ends of the specimen 1. Each sample-holder 2 comprises a vice 3 which is made to clamp the specimen 1 and is provided with two jaws 4 and 5, which engage opposite sides of the specimen 1. In each sample-holder 2 the two jaws 4 and 5 of the vice 3 are crossed and mutually connected by means of a screw 6: by screwing the screw 6 the two jaws 4 and 5 are approached to one another (thus clamping the specimen 1) while unscrewing the screw 6 the two jaws 4 and 5 are distanced from one another (thus releasing the specimen 1). In each sample-holder 2, the jaw 4 of the vice 3 is provided with an extension 7 that projects itself from the jaw 4, terminates with a frusto-conical head and is used both to *"park"* the specimen 1 assembled with two sample-holders 2 in a warehouse and to mechanically connect the sample-holder 2 to the active members of a measuring machine (known and not shown), which carries out the dynamic mechanical-thermal analysis.

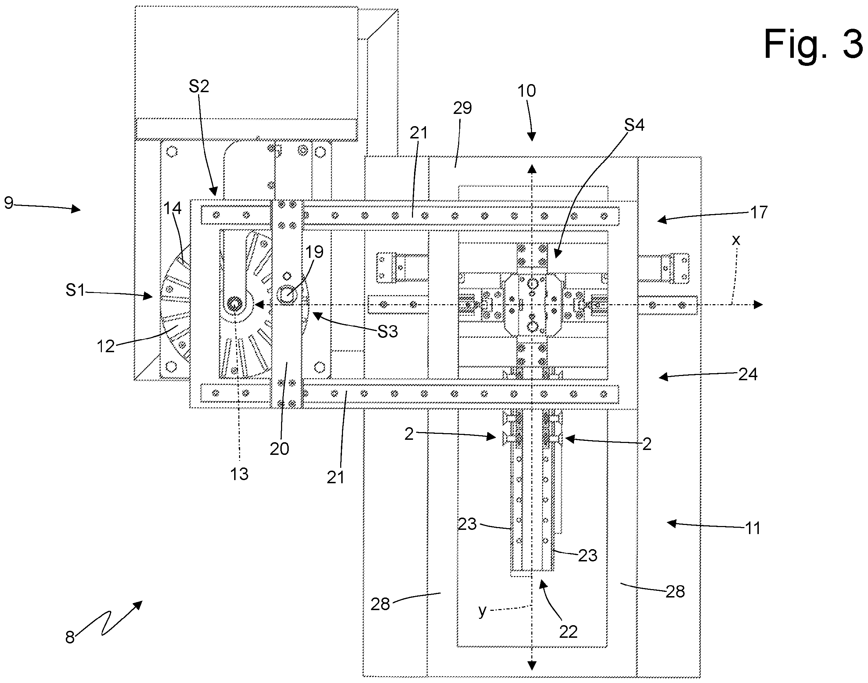

In Figure 3, number 8 denotes as a whole an assembling machine that receives in input the specimens 1, carries out at least one measurement on each specimen 1 (in particular it measures at least the thickness of each specimen 1), and couples to each specimen 1 a pair of sample-holders 2.

As shown in Figure 3, the assembling machine 8 comprises a measuring unit 9 which receives the specimens 1 from the outside and carries out a measurement of the specimens 1 themselves, a mounting unit 10 which receives the specimens 1 from the measuring unit 9 and applies to each specimen 1 two corresponding sample-holders 2, and a storage unit 11 which receives the specimens 1 coupled to the sample-holders 2 from the assembling unit 10 and stores ("*parks*") the specimens 1 themselves until they are collected and analyzed by the measuring machine 1.

The measuring unit 9 comprises a wheel (carousel) 12 which is mounted so as to rotate with intermittent motion (i.e. with a stepped motion which cyclically alternates stoppage phases and motion phases) around a vertical rotation axis 13. The wheel 12 presents a plurality of pockets 14 (twenty pockets 14 in the non-limiting embodiment illustrated in the annexed figures), which are oriented radially and are each made to contain a corresponding specimen 1. The rotation of the wheel 12 around the axis 13 of rotation advances cyclically each pocket 14 through: an input station S1 wherein an operator inserts inside the pocket 14 a specimen 1, a measuring station S2 wherein the thickness of the specimen 1 contained in the pocket 14 is measured, and an output station S3 wherein the specimen 1 is extracted from the pocket 14 and transferred to the assembling unit 10.

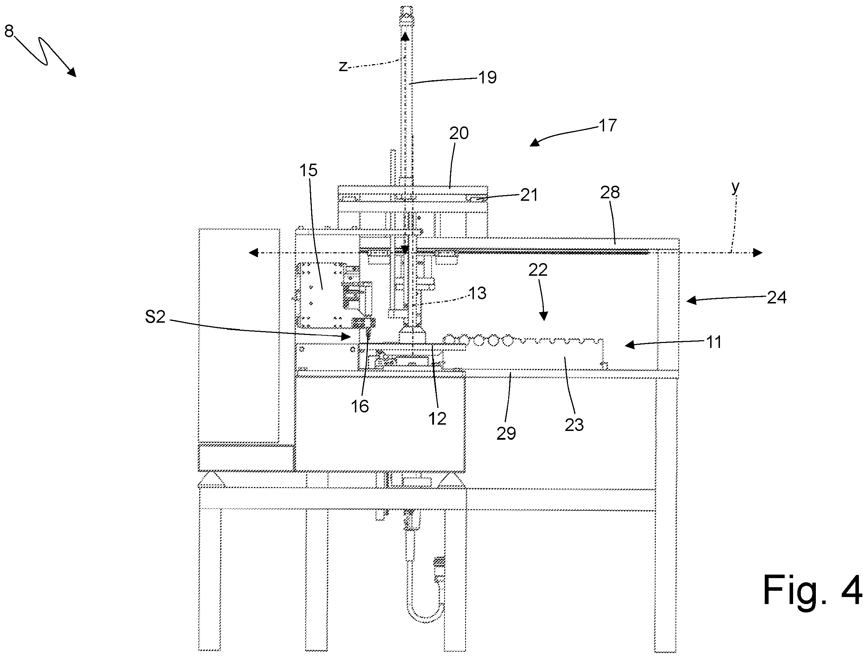

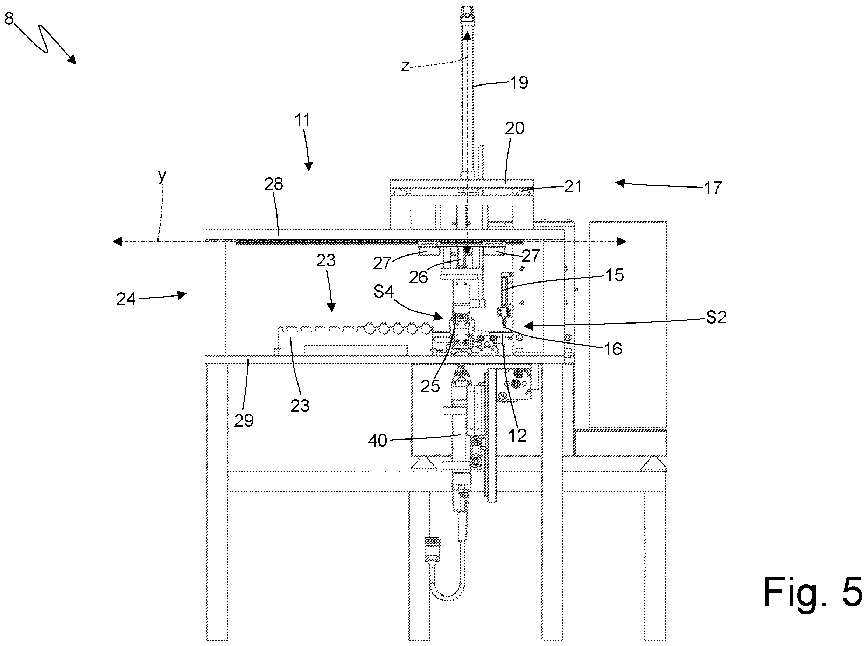

As shown in Figures 4 and 5, the measuring unit 9 comprises a measuring device 15, which is located at the measuring station S2 and comprises at least a sensor 16 that measures the thickness of a specimen 1 in at least three distinct points.

As shown in Figures 3-6, the mounting unit 10 comprises a transfer device 17 which cyclically collects the specimens 1 from the pockets 14 of the wheel 12 in the output station S3 and cyclically deposits the specimens 1 in a processing station S4 of the mounting unit 10. The transfer device 17 is of the *"portal"* type and comprises a suction gripping head 18 that is moved (at least) with two degrees of freedom along a vertical direction z and along a longitudinal direction x (arranged horizontally). In particular, the suction gripping head 18 is carried by an actuating cylinder 19 (for example pneumatic or electric), which is arranged vertically and is made to move the suction gripping head 18 vertically (i.e. along the vertical direction z); the actuating cylinder 19 is mounted on a slide 20 which slides (for example due to the action of an electric motor) along two rails 21 parallel to one another which are arranged above the wheel 12 and above the processing station S4.

As illustrated in Figures 3, 4 and 5, the storage unit 11 comprises (at least) a rack 22, which comprises two supports 23 that are parallel to one another and arranged side by side and have respective successions of semicircular recesses made to contain (receive) the cylindrical portions of the extensions 7 of the sample-holders 2. The rack 22 is made to contain both the empty sample-holders 2 (i.e. without specimens 1 and therefore ready to be coupled to the specimens 1) and the full sample-holders 2 (i.e. coupled to the respective specimens 1).

As shown in Figures 3-7, the mounting unit 10 comprises a (further) transfer device 24, which collects a pair of empty sample-holders 2 (i.e. without specimens 1) from the rack 22 of the storage unit 11 and deposits the pair of empty sample-holders 2 (i.e. without specimens 1) in the processing station S4 of the mounting unit 10, wherein the pair of sample-holders 2 is coupled to a specimen 1; subsequently, the transfer device 24 collects the pair of full sample-holders 2 (i.e. coupled to a respective specimen 1) from the processing station S4 of the mounting unit 10 and deposits the pair of full sample-holders 2 (i.e. coupled to a respective specimen 1) in the rack 22 of the storage unit 11.

The transfer device 24 is of the *"portal"* type and comprises a pair of grippers 25, which are arranged parallel to each other and spaced apart and each of which is made to grip (and therefore retain with force) the cylindrical portion of an extension 7 of a sample-holder 2; the pair of grippers 25 is moved (at least) with two degrees of freedom along the vertical direction z and along a transversal direction Y (arranged horizontally and illustrated in Figures 3-5). In particular, the pair of grippers 25 is carried by an actuating cylinder 26 (for example pneumatic or electric), which is arranged vertically and is made to move the pair of grippers 25 vertically (i.e. along the vertical direction z); the actuating cylinder 26 is mounted on a pair of slides 27 which slide (for example due to the action of an electric motor) along two rails 28 (shown in Figure 1) parallel to each other which are arranged above the rack 22 and above the processing station S4 but lower than the rails 21.

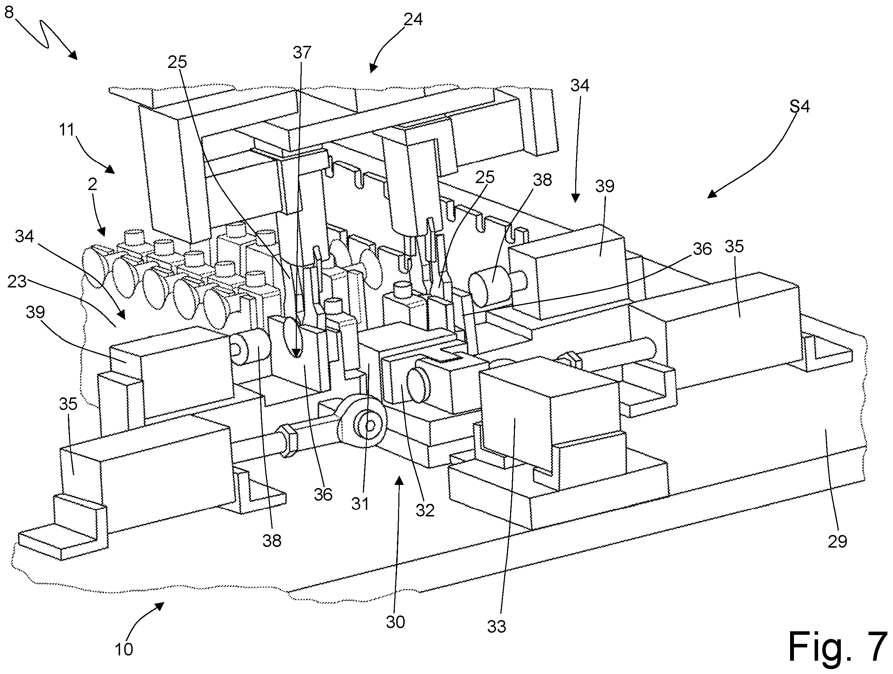

As shown in Figure 7, the processing station S4 of the mounting unit 10 comprises a central working surface 29 arranged horizontally and on which a central vise 30 is mounted in a fixed position which is made to receive and tighten a specimen 4. The central vise 30 includes a fixed jaw 31 (i.e. that does not move), which establishes a reference position, and a movable jaw 32, which is parallel and opposite to the fixed jaw 31 and is movably mounted under the thrust of an actuator 33 (for example a pneumatic or an electric cylinder). In use, the actuator 33 moves the movable jaw 32 away from the fixed jaw 31 to allow the transfer device 24 to insert a specimen 1 between the two jaws 31 and 32 and then the actuator 33 approaches the movable jaw 32 to the fixed jaw 31 to clamp between the two jaws 31 and 32 a central portion of the specimen 1 and, at the same time, arrange the specimen 1 in a given and predetermined position established by the fixed jaw 31.

As shown in Figure 7, the processing station S4 of the mounting unit 10 includes a pair of movable elements 34, which are arranged on the opposite sides of the central vice 30 and are mounted on respective rails fixed to the working surface 29 to slide (translate) only and solely along the longitudinal direction x (i.e. along a straight horizontal direction) under the control of two corresponding actuators 35 (for example pneumatic or electric cylinders); in use, the two movable elements 34 are moved away or closer together and in a specular manner to the central vice 30, which is arranged between the two movable elements 34 themselves. Each movable element 34 supports a supporting plate 36 which is made to receive and accommodate a corresponding sample-holder 2; in particular the supporting plate 36 comprises a semicircular recess 37 made to contain (receive) the frusto-conical portion of the extension 7 of a sample-holder 2 (normally the semicircular recess 37 negatively reproduces the frusto-conical shape of the extension 7 and therefore also has a frusto-conical shape). In addition, each movable element 34 supports a presser 38 which is arranged opposite the recess 37 of the supporting plate 36 and is movable along the longitudinal direction x under the control of an actuator 39 (for example a pneumatic or an electric cylinder).

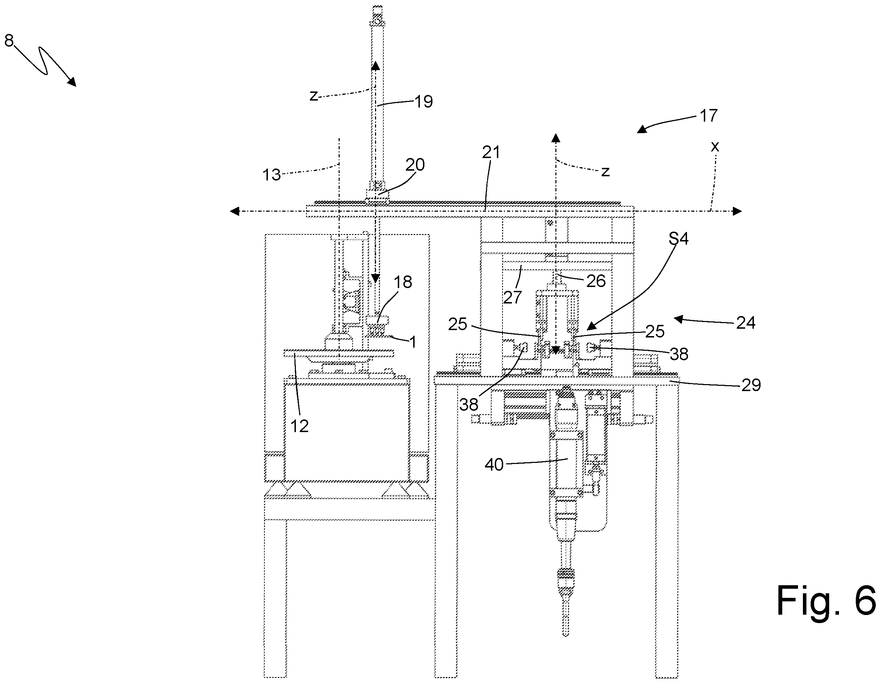

Finally, the processing station 10 comprises a motorized screwing device 40 (illustrated in Figures 5 and 6), which is arranged below the working surface 29 and is made to screw/unscrew the screws 6 of the sample-holders 2 housed in the supporting plates 36 to open or close the vices 6 of the sample-holders 2 themselves.

A description is given below of the operation of the assembling machine 8 with reference to the processing of a single specimen 1.

Initially the specimen 1 is inserted manually by an operator in a pocket 14 of the wheel 12 in the input station S1.

Subsequently the rotation of wheel 12 around the rotation axis 13 moves the pocket 14 carrying the specimen 1 from the input station S1 to the measuring station S2 wherein the sensor 16 of the measuring device 15 detect the thickness of the specimen 1 in at least three distinct zones of the specimen 1 itself.

Subsequently the rotation of the wheel 12 around the axis of rotation 13 moves the pocket 14 carrying the specimen 1 from the measuring station S2 to the output station S3 wherein the suction gripping head 18 of the transfer device 17 collects the specimen 1 from the pocket 14 of the wheel 12 and transfers the specimen 1 to the processing station S4 of the mounting unit 10; in particular, in the processing station S4, the transfer device 17 deposits the specimen inside the central vice 30 whose jaws 31 and 32 have been suitably distanced from one another by the actuator 33 (i.e. the central vice 30 has been opened for a smooth entry of the specimen 1). Once the specimen 1 has been inserted in the central vice 30 (i.e. between the two jaws 31 and 32 of the central vice 30), the central vice 30 is closed by the actuator 33 (i.e. the movable jaw 32 of the central vice 30 is pushed with a calibrated force toward the fixed jaw 31 to lock the specimen 1 in a predetermined position).

Simultaneously with the feeding of the specimen 1 to the central vice 30, the two grippers 25 of the transfer device 24 collect two empty sample-holders 2 (i.e. without specimens 1) from the rack 22 of the storage unit 11 and deposit the two empty sample-holders 2 onto the supporting plates 36 of the movable elements 34; in this phase the two movable elements 34 are arranged and maintained (relatively) away from the central vise 30 in such a way that the empty sample-holders 2 carried by the supporting plates 36 do not interfere with the specimen 1 carried by the central vice 30. It is important to observe that the empty sample-holders 2 (i.e. without specimens 1) arranged on the rack 22 of the storing unit 11 have their respective vices 3 fully open, i.e. the jaws 4 and 5 of the respective vices 3 are distant from one another in such a way as to be ready to receive the ends of a specimen 1. Once the two sample-holders 2 have been arranged on the supporting plates 36, the actuators 39 push the two pressers 38 against the ends of the projections 7 of the two sample-holders 2 to push the extensions 7 themselves against the recesses 37 of the supporting plates 36 and then arrange the extensions 7 (therefore the corresponding sample-holders 2) in a given predetermined position established by the recesses 37.

At this point, the actuators 35 approach the two movable elements 34 to the central vice 30 (in this phase the actuators 39 maintain the pressers 38 pressed against the ends of the extensions 7 of the two sample-holders 2 to keep the two sample-holders 2 in a given predetermined position established by the recesses 37) in such a way that the two ends of the specimen 1 carried by the central vice 30 enter into the vices 3 of the two sample-holders 2 carried by the supporting plates 36 of the movable elements 34 (i.e. in such a way that the vices 3 of the two sample-holders 2 carried by the supporting plates 36 of the movable elements 34 are arranged around the two ends of the specimen 1 carried by the central vice 30). Once the approach stroke of the movable elements 34 to the central vice 30 has ended (i.e. when the movable elements 34 stops because the two ends of the specimen 1 carried by the central vice 30 are completely inside the vices 3 of the two sample-holders 2 carried by the supporting plates 36 of the movable elements 34), the screwing device 40 is activated and acts on the screws 6 of the vices 3 of the two sample-holders 2 carried by the supporting plates 36 of the movable elements 34 in such a way as to close (tighten) with a predetermined clamping force of the vices 3 of the two sample-holders 2 against the ends of the specimen 1 carried by the central vice 30. In this respect it is important to observe that the screwing device 40 is preferably of the dynamometric type to apply to the vices 3 of the two sample-holders 2 a calibrated and predetermined tightening force.

Subsequently, i.e. once the vices 3 of the two sample-holders 2 have been tightened against the ends of the specimen 1 carried by the central vice 30, actuators 39 pull away the pressers 38 from the extensions 7 of the two sample-holders 2 carried by the supporting plates 36 and then the two grippers 25 of the transfer device 24 collect the two full sample-holders 2 (i.e. coupled to the specimen 1) from the supporting plates 36 of the two movable elements 34 and deposit the two full sample-holders 2 (i.e. coupled to the specimen 1) on the rack 22 of the storage unit 11.

The assembling machine 8 described above has many advantages.

Firstly, the assembling machine 8 described above makes it possible to significantly reduce the time required to prepare the specimens 1 for a dynamic mechanical-thermal analysis: in the assembling machine 8 the time required for the preparation of a specimen 1 is approximately one minute while a laboratory operator requires on average over four minutes to carry out the same operation (i.e. a quadruple time).

In addition, the assembling machine 8 described above provides a net improvement in the repeatability of the analysis by eliminating all possible accidental errors which may be committed by a laboratory operator (particularly, but not only, in checking the alignment of the specimen 1 with respect to the two sample-holders 2 and in controlling the tightening force of the vices 3 of the two sample-holders 2).

### LIST OF REFERENCE NUMBERS IN THE FIGURES

- 1: specimen
- 2: sample-holder
- 3: vice
- 4: jaw
- 5: jaw
- 6: screw
- 7: extension
- 8: assembly machine
- 9: measuring unit
- 10: mounting unit
- 11: storage unit
- 12: wheel
- 13: rotation axis
- 14: pockets
- 15: measuring device
- 16: sensors
- 17: transfer device
- 18: gripping head
- 19: actuating cylinder
- 20: slide
- 21: rails
- 22: rack
- 23: supports
- 24: transfer device
- 25: grippers
- 26: actuating cylinder
- 27: slide
- 28: rails
- 29: working surface
- 30: central vice
- 31: fixed jaw
- 32: movable jaw
- 33: actuator
- 34: movable elements
- 35: actuators
- 36: support plates
- 37: recess
- 38: presser
- 39: actuator
- 40: screwing device
- S1: input station
- S2: measuring station
- S3: output station
- S4: processing station
- x: longitudinal direction
- y: transverse direction
- z: vertical direction

## Claims

1. Assembling machine (8) for coupling the ends of an elastomer specimen (1) to be subjected to dynamic mechanical-thermal analysis to two sample-holders (2), each sample-holder comprising a vice (3) which is made to clamp the specimen (1); the assembling machine (8) **characterized in that** it comprises:
a horizontal working surface (29);
a central vice (30) which is mounted in fixed position on the horizontal working surface (29) and is made to engage a central portion of the specimen (1);
two movable elements (34), which are mounted on the horizontal working surface (29), are arranged on the opposite sides of the central vice (30), and each carry a supporting plate (36) made to accommodate a corresponding sample-holder (2);
a first actuator (35) that is made to translate the two movable elements (34) only and solely along a straight horizontal direction to move in/out the two movable elements (34) from/to the central vice (30); and
a powered screwing device (40) which is disposed in the vicinity of the central vice (30) and is made for screwing/unscrewing a screw (6) of the vice (3) of each sample-holder (2) to close the vice (3) itself against an end of the sample (1) carried by the central vice (30).

2. Assembling machine (8) according to claim 1, wherein the screwing device (40) is arranged below the horizontal working surface (29).

3. Assembling machine (8) according to claim 1 or 2, wherein the central vice (30) comprises a fixed jaw (31) which establishes a reference position and a movable jaw (32) which is parallel and opposite to the fixed jaw (31) and is movably mounted under the thrust of a second actuator (33).

4. Assembling machine (8) according to claim 1, 2 or 3, wherein the two movable elements (34) are mounted on respective rails fixed to slide under the control of two corresponding first actuators (35).

5. Assembling machine (8) according to one of the claims 1 to 4, wherein each supporting plate (36) comprises a semicircular recess (37) made to contain a frusto-conical portion of an extension (7) of a sample-holder (2).

6. Assembling machine (8) according to claim 5, wherein each movable element (34) supports a presser (38) which is arranged in front of the recess (37) of the supporting plate (36) and is movable under the control of a third actuator (39) to push the frusto-conical portion of the extension (7) of a sample-holder (2) against the recess (37) of the supporting plate (36).

7. Assembling machine (8) according to one of the claims 1 to 6 and comprising:
a storage unit (11) which stores the empty sample-holders (2) and the sample-holders (2) coupled to the specimens (1); and
a first transfer device (24) which is made to transfer a pair of empty sample-holders (2) from the storage unit (11) to the two supporting plates (36) and to transfer a pair of sample-holders (2) coupled to a specimen (1) from the two supporting plates (36) to the storage unit (11).

8. Assembling machine (8) according to claim 7, wherein the storage unit (11) comprises at least one rack (22), which comprises two supports (23) which are parallel to each other and arranged side by side and have respective successions of semicircular recesses made to contain cylindrical portions of extensions (7) of the sample-holders (2).

9. Assembling machine (8) according to claim 7 or 8, wherein the first transfer device (24) comprises a pair of grippers (25), which are arranged parallel to each other and spaced apart and each of which is made to grip a cylindrical portion of an extension (7) of a sample-holder (2).

10. Assembling machine (8) according to one of the claims 1 to 9 and comprising:
a measuring unit (9) which receives the specimens (1) from the outside and carries out a measurement of the specimens (1) themselves; and
a second transfer device (17), which transfers the specimen (1) from the measuring unit (9) to the central vice (30).

11. The assembling machine (8) according to claim 10, wherein the measuring unit (9) comprises a wheel (12) which is rotatably mounted so as to rotate with an intermittent motion about a vertical rotation axis (13) and has a plurality of pockets (14), which are orientated radially and are each made to contain a corresponding specimen (1).

12. Assembling machine (8) according to claim 11, wherein the rotation of the wheel (12) around the rotation axis (13) advances cyclically each pocket (14) through: an input station (51) wherein a specimen (1) is inserted inside the pocket (14), a measuring station (S2) wherein the thickness of the specimen (1) contained in the pocket (14) is measured, and an output station (S3) wherein the specimen (1) is extracted from the pocket (14) and transferred to the central vice (30) by the second transfer device (17).

13. Assembling machine (8) according to claim 12, wherein the measurement unit (9) comprises a measuring device (15), which is arranged in correspondence of the measurement station (S2) and comprises at least one sensor (16) which measures the thickness of a specimen (1) in at least three distinct points.

14. Assembly method for coupling the ends of an elastomer specimen (1) to be subjected to dynamic mechanical-thermal analysis to two-sample-holders (2) each sample-holder comprising a vice (3) which is made to clamp the specimen (1); the assembly method **characterized in that** it comprises the steps of:
engaging a central portion of the specimen (1) by means of a central vice (30) mounted in a fixed position on a horizontal working surface (29);
having two sample-holders (2) on corresponding supporting plates (36) carried by two movable elements (34), which are mounted on the horizontal working surface (29) and are arranged on opposite sides of the central vice (30);
bringing the two movable elements (34) towards the central vice (30) by the action of a first actuator (35) which translates the two movable elements (34) only and solely along a straight horizontal direction; and
closing a vice (3) of each sample-holder (2) against an end of the specimen (1) carried by the central vice (30) using a motorized screwing device (40) which is arranged in the vicinity of the central vice (30) and is made to screw/unscrew a screw (6) of the vice (3) of the sample-holder (2).

15. Assembly method according to claim 14, wherein:
each supporting plate (36) comprises a semicircular recess (37) made to contain a frusto-conical portion of an extension (7) of a sample-holder (2); and
each movable device (34) supports a presser (38) which is arranged in front of the recess (37) of the supporting plate (36) and is movable under the control of a second actuator (39) to push the frusto-conical portion of the extension (7) of a sample-holder (2) against the recess (37) of the supporting plate (36).

## Patentansprüche

1. Montagemaschine (8) zum Verbinden der Enden eines Elastomermusters (1), das einer dynamischen mechanisch-thermischen Analyse unterzogen werden soll, mit zwei Probenhaltern (2), jeder Probenhalter umfassend eine Schraubzwinge (3), die gefertigt ist, um das Muster (1) zu klemmen; wobei die Montagemaschine (8) **dadurch gekennzeichnet ist, dass** sie umfasst:
eine horizontale Arbeitsoberfläche (29);
eine mittlere Schraubzwinge (30), die in einer festen Position auf der horizontalen Arbeitsoberfläche (29) angebracht und gefertigt ist, um in einen mittleren Abschnitt des Musters (1) einzugreifen;
zwei bewegbare Elemente (34), die auf der horizontalen Arbeitsoberfläche (29) angebracht sind, auf den gegenüberliegenden Seiten der mittleren Schraubzwinge (30) angeordnet sind und jeweils eine Stützplatte (36) tragen, die gefertigt ist, um einen entsprechenden Probenhalter (2) aufzunehmen;
einen ersten Aktor (35), der gefertigt ist, um die zwei bewegbaren Elemente (34) nur und ausschließlich entlang einer geraden horizontalen Richtung zu verschieben, um die zwei bewegbaren Elemente (34) aus der/in die mittlere Schraubzwinge (30) heraus/hinein zu bewegen; und
eine angetriebene Schraubvorrichtung (40), die in der Nähe der mittleren Schraubzwinge (30) eingerichtet ist und zum Ein-/Herausschrauben einer Schraube (6) der Schraubzwinge (3) jedes Probenhalters (2) gefertigt ist, um die Schraubzwinge (3) selbst gegen ein Ende des Musters (1) zu schließen, das durch die mittlere Schraubzwinge (30) getragen wird.

2. Montagemaschine (8) nach Anspruch 1, wobei die Schraubvorrichtung (40) unterhalb der horizontalen Arbeitsoberfläche (29) angeordnet ist.

3. Montagemaschine (8) nach Anspruch 1 oder 2, wobei die mittlere Schraubzwinge (30) eine feste Backe (31), die eine Referenzposition festlegt, und eine bewegbare Backe (32) umfasst, die parallel und gegenüber der festen Backe (31) liegt, und unter dem Schub eines zweiten Aktors (33) bewegbar angebracht ist.

4. Montagemaschine (8) nach Anspruch 1, 2 oder 3, wobei die zwei bewegbaren Elemente (34) auf den jeweiligen Schienen angebracht sind, die befestigt sind, um unter der Steuerung von zwei entsprechenden ersten Aktoren (35) zu gleiten.

5. Montagemaschine (8) nach einem der Ansprüche 1 bis 4, wobei jede Stützplatte (36) eine halbkreisförmige Aussparung (37) umfasst, die gefertigt ist, um einen kegelstumpfförmigen Abschnitt einer Verlängerung (7) eines Probenhalters (2) zu enthalten.

6. Montagemaschine (8) nach Anspruch 5, wobei jedes bewegbare Element (34) einen Presser (38) stützt, der vor der Aussparung (37) der Stützplatte (36) angeordnet ist und unter der Steuerung eines dritten Aktors (39) bewegbar ist, um den kegelstumpfförmigen Abschnitt der Verlängerung (7) eines Probenhalters (2) gegen die Aussparung (37) der Stützplatte (36) zu drücken.

7. Montagemaschine (8) nach einem der Ansprüche 1 bis 6 und umfassend:
eine Lagereinheit (11), die die leeren Probenhalter (2) und die mit den Mustern (1) gekoppelten Probenhalter (2) lagert; und
eine erste Überführungsvorrichtung (24), die gefertigt ist, um ein Paar von leeren Probenhaltern (2) von der Lagereinheit (11) zu den zwei Stützplatten (36) zu überführen und ein Paar Probenhalter (2), die mit einem Muster (1) verbunden sind, von den zwei Stützplatten (36) zu der Lagereinheit (11) zu überführen.

8. Montagemaschine (8) nach Anspruch 7, wobei die Lagereinheit (11) mindestens ein Gestell (22) umfasst, das zwei Stützen (23) umfasst, die parallel zueinander und nebeneinander angeordnet sind und jeweilige aufeinanderfolgende Reihen halbkreisförmiger Aussparungen aufweisen, die gefertigt sind, um zylindrische Abschnitte von Verlängerungen (7) der Probenhalter (2) zu enthalten.

9. Montagemaschine (8) nach Anspruch 7 oder 8, wobei die erste Überführungsvorrichtung (24) ein Paar von Greifern (25) umfasst, die parallel und beabstandet zueinander angeordnet und jeweils gefertigt sind, um einen zylindrischen Abschnitt einer Verlängerung (7) eines Probenhalters (2) zu greifen.

10. Montagemaschine (8) nach einem der Ansprüche 1 bis 9 und umfassend:
eine Messeinheit (9), die die Muster (1) von außen empfängt und eine Messung der Muster (1) selbst durchführt; und
eine zweite Überführungsvorrichtung (17), die die Muster (1) von der Messeinheit (9) zu der mittleren Schraubzwinge (30) überführt.

11. Montagemaschine (8) nach Anspruch 10, wobei die Messeinheit (9) ein Rad (12) umfasst, das drehbar angebracht ist, um mit einer intermittierenden Bewegung um eine vertikale Drehachse (13) zu drehen, und das eine Vielzahl von Taschen (14) aufweist, die radial ausgerichtet sind und jeweils gefertigt ist, um ein entsprechendes Muster (1) zu enthalten.

12. Montagemaschine (8) nach Anspruch 11, wobei die Drehung des Rads (12) um die Drehachse (13) herum jede Tasche (14) zyklisch vorwärts bewegt über: eine Eingabestation (S1), in der ein Muster (1) in ein Inneres der Tasche (14) eingeführt wird, eine Messstation (S2), in der die Dicke des Musters (1), das in der Tasche (14) enthalten ist, gemessen wird, und eine Ausgabestation (S3), in der das Muster (1) aus der Tasche (14) gezogen und durch die zweite Überführungsvorrichtung (17) zu der mittleren Schraubzwinge (30) überführt wird.

13. Montagemaschine (8) nach Anspruch 12, wobei die Messeinheit (9) eine Messvorrichtung (15) umfasst, die entsprechend der Messstation (S2) angeordnet ist und mindestens einen Sensor (16) umfasst, der die Dicke eines Musters (1) an mindestens drei verschiedenen Punkten misst.

14. Montageverfahren zum Verbinden der Enden eines Elastomermusters (1), das einer dynamischen mechanisch-thermischen Analyse unterzogen werden soll, mit zwei Probenhaltern (2), jeder Probenhalter umfassend eine Schraubzwinge (3), die gefertigt ist, um das Muster (1) zu klemmen; wobei das Montageverfahren **dadurch gekennzeichnet ist, dass** es die Schritte umfasst:
Eingreifen eines mittleren Abschnitts des Musters (1) mittels einer mittleren Schraubzwinge (30), die in einer festen Position auf einer horizontalen Arbeitsoberfläche (29) angebracht ist;
Aufweisen von zwei Probenhaltern (2) auf den entsprechenden Stützplatten (36), die durch zwei bewegbare Elemente (34) getragen werden, die auf der horizontalen Arbeitsoberfläche (29) angebracht und auf den gegenüberliegenden Seiten der mittleren Schraubzwinge (30) angeordnet sind;
Bringen der zwei bewegbaren Elemente (34) zu der mittleren Schraubzwinge (30) hin durch die Wirkung eines ersten Aktors (35), der die zwei bewegbaren Elemente (34) nur und ausschließlich entlang einer geraden horizontalen Richtung bewegt; und
Schließen einer Schraubzwinge (3) jedes Probenhalters (2) gegen ein Ende des Musters (1), das durch die mittlere Schraubzwinge (30) getragen wird, unter Verwendung einer motorisierten Schraubvorrichtung (40), die in der Nähe der mittleren Schraubzwinge (30) angeordnet und gefertigt ist, um eine Schraube (6) der Schraubzwinge (3) des Probenhalters (2) einzuschrauben/herauszuschrauben.

15. Montageverfahren nach Anspruch 14, wobei:
jede Stützplatte (36) eine halbkreisförmige Aussparung (37) umfasst, die gefertigt ist, um einen kegelstumpfförmigen Abschnitt einer Verlängerung (7) eines Probenhalters (2) zu enthalten; und
jedes bewegbare Element (34) einen Presser (38) stützt, der vor der Aussparung (37) der Stützplatte (36) angeordnet ist und unter der Steuerung eines zweiten Aktors (39) bewegbar ist, um den kegelstumpfförmigen Abschnitt der Verlängerung (7) eines Probenhalters (2) gegen die Aussparung (37) der Stützplatte (36) zu drücken.

## Revendications

1. Machine d'assemblage (8) destinée à accoupler les extrémités d'un échantillon d'élastomère (1) à soumettre à une analyse mécanique-thermique dynamique à deux porte-échantillons (2), chaque porte-échantillon comprenant un étau (3) qui est fait pour serrer l'échantillon (1) ; la machine d'assemblage (8) est **caractérisée en ce qu'elle** comprend :
une surface de travail horizontale (29) ;
un étau central (30) qui est monté en position fixe sur la surface de travail horizontale (29) et est fait pour venir en prise avec une partie centrale de l'échantillon (1) ;
deux éléments mobiles (34), qui sont montés sur la surface de travail horizontale (29), sont agencés sur les côtés opposés de l'étau central (30), et portent chacun une plaque de support (36) faite pour loger un porte-échantillon (2) correspondant ;
un premier actionneur (35) qui est fait pour effectuer une translation des deux éléments mobiles (34) uniquement et seulement le long d'une direction horizontale linéaire pour déplacer dans/hors les deux éléments mobiles (34) de/vers l'étau central (30) ; et
un dispositif de vissage alimenté (40) qui est disposé à proximité de l'étau central (30) et est fait pour le vissage/dévissage d'une vis (6) de l'étau (3) de chaque porte-échantillon (2) pour fermer l'étau (3) lui-même contre une extrémité de l'échantillon (1) porté par l'étau central (30).

2. Machine d'assemblage (8) selon la revendication 1, dans laquelle le dispositif de vissage (40) est agencé sous la surface de travail horizontale (29).

3. Machine d'assemblage (8) selon la revendication 1 ou 2, dans laquelle l'étau central (30) comprend une mâchoire fixe (31) qui établit une position de référence et une mâchoire mobile (32) qui est parallèle et à l'opposée de la mâchoire fixe (31) et est montée de manière mobile au-dessous de la poussée d'un deuxième actionneur (33).

4. Machine d'assemblage (8) selon la revendication 1, 2 ou 3, dans laquelle les deux éléments mobiles (34) sont montés sur des rails respectifs fixés pour coulisser sous la commande de deux premiers actionneurs (35) correspondants.

5. Machine d'assemblage (8) selon l'une des revendications 1 à 4, dans laquelle chaque plaque de support (36) comprend un évidement semi-circulaire (37) fait pour contenir une partie tronconique d'une extension (7) d'un porte-échantillon (2).

6. Machine d'assemblage (8) selon la revendication 5, dans laquelle chaque élément mobile (34) supporte un presseur (38) qui est agencé devant l'évidement (37) de la plaque de support (36) et est mobile sous la commande d'un troisième actionneur (39) pour pousser la partie tronconique de l'extension (7) d'un porte-échantillon (2) contre l'évidement (37) de la plaque de support (36).

7. Machine d'assemblage (8) selon l'une des revendications 1 à 6 et comprenant :
une unité de stockage (11) qui stocke les porte-échantillons (2) vides et les porte-échantillons (2) accouplés aux échantillons (1) ; et
un premier dispositif de transfert (24) qui est fait pour transférer une paire de porte-échantillons (2) vides de l'unité de stockage (11) aux deux plaques de support (36) et pour transférer une paire de porte-échantillons (2) accouplés à un spécimen (1) des deux plaques de support (36) à l'unité de stockage (11).

8. Machine d'assemblage (8) selon la revendication 7, dans laquelle l'unité de stockage (11) comprend au moins un présentoir (22), qui comprend deux supports (23) qui sont parallèles l'un à l'autre et agencés côte à côte et ont des successions respectives d'évidements semi-circulaires faits pour contenir des parties cylindriques d'extensions (7) des porte-échantillons (2).

9. Machine d'assemblage (8) selon la revendication 7 ou 8, dans laquelle le premier dispositif de transfert (24) comprend une paire d'organes de préhension (25), qui sont agencés parallèles l'un à l'autre et espacés et dont chacun est fait pour saisir une partie cylindrique d'une extension (7) d'un porte-échantillon (2).

10. Machine d'assemblage (8) selon l'une des revendications 1 à 9 et comprenant :
une unité de mesure (9) qui reçoit les échantillons (1) de l'extérieur et réalise une mesure des échantillons (1) eux-mêmes ; et
un second dispositif de transfert (17), qui transfère l'échantillon (1) de l'unité de mesure (9) à l'étau central (30).

11. Machine d'assemblage (8) selon la revendication 10, dans laquelle l'unité de mesure (9) comprend une roue (12) qui est montée de manière rotative de façon à entrer en rotation avec un mouvement intermittent autour d'un axe de rotation vertical (13) et a une pluralité de poches (14), qui sont orientées de manière radiale et sont chacune faites pour contenir un échantillon (1) correspondant.

12. Machine d'assemblage (8) selon la revendication 11, dans laquelle la rotation de la roue (12) autour de l'axe de rotation (13) avance de manière cyclique chaque poche (14) à travers : une station d'entrée (S1) dans laquelle un échantillon (1) est inséré à l'intérieur de la poche (14), une station de mesure (S2) dans laquelle l'épaisseur de l'échantillon (1) contenu dans la poche (14) est mesurée, et une station de sortie (S3) dans laquelle l'échantillon (1) est extrait de la poche (14) et transféré à l'étau central (30) par le second dispositif de transfert (17).

13. Machine d'assemblage (8) selon la revendication 12, dans laquelle l'unité de mesure (9) comprend un dispositif de mesure (15), qui est agencé en correspondance de la station de mesure (S2) et comprend au moins un capteur (16) qui mesure l'épaisseur d'un échantillon (1) en au moins trois points distincts.

14. Procédé d'assemblage destiné à accoupler les extrémités d'un échantillon d'élastomère (1) à soumettre à une analyse mécanique-thermique dynamique à deux porte-échantillons (2), chaque porte-échantillon comprenant un étau (3) qui est fait pour serrer l'échantillon (1) ; le procédé d'assemblage est **caractérisé en ce qu'**il comprend les étapes consistant à :
amener en prise une partie centrale de l'échantillon (1) au moyen d'un étau central (30) monté dans une position fixe sur une surface de travail horizontale (29) ;
avoir deux porte-échantillons (2) sur des plaques de support (36) correspondantes portées par deux éléments mobiles (34), qui sont montés sur la surface de travail horizontale (29) et sont agencés sur des côtés opposés de l'étau central (30) ;
amener les deux éléments mobiles (34) vers l'étau central (30) par l'action d'un premier actionneur (35) qui effectue une translation des deux éléments mobiles (34) uniquement et seulement le long d'une direction horizontale linéaire ; et
fermer un étau (3) de chaque porte-échantillon (2) contre une extrémité de l'échantillon (1) porté par l'étau central (30) au moyen d'un dispositif de vissage motorisé (40) qui est agencé à proximité de l'étau central (30) et est fait pour visser/dévisser une vis (6) de l'étau (3) du porte-échantillon (2).

15. Procédé d'assemblage selon la revendication 14, dans lequel :
chaque plaque de support (36) comprend un évidement semi-circulaire (37) fait pour contenir une partie tronconique d'une extension (7) d'un porte-échantillon (2) ; et
chaque dispositif mobile (34) supporte un presseur (38) qui est agencé devant l'évidement (37) de la plaque de support (36) et est mobile sous la commande d'un deuxième actionneur (39) pour pousser la partie tronconique de l'extension (7) d'un porte-échantillon (2) contre l'évidement (37) de la plaque de support (36).
